(19) Europäisches Patentamt
European Patent Office
Office européen des brevets

(11) Veröffentlichungsnummer: **0 254 219**
**B1**

(12) # EUROPÄISCHE PATENTSCHRIFT

(45) Veröffentlichungstag der Patentschrift: **22.11.90**

(21) Anmeldenummer: **87110348.7**

(22) Anmeldetag: **17.07.87**

(51) Int. Cl.[5]: **C 07 C 33/46**, C 07 C 29/80, C 07 C 29/38

(54) **Verfahren zur Trennung von Isomerengemischen aus 1.3- und 1.4-Bis-(2-hydroxyhexafluoro-2-propyl)-benzol.**

(30) Priorität: **23.07.86 DE 3624911**

(43) Veröffentlichungstag der Anmeldung: **27.01.88 Patentblatt 88/04**

(45) Bekanntmachung des Hinweises auf die Patenterteilung: **22.11.90 Patentblatt 90/47**

(84) Benannte Vertragsstaaten: **BE CH DE FR GB IT LI NL**

(56) Entgegenhaltungen:
**US-A-3 236 894**
**US-A-3 304 334**

**THE JOURNAL OF ORGANIC CHEMISTRY, Band 30, 1965, Easton, Pennsylvania, USA; B. S. FARAH et al. "Perhalo Ketones. V. The Reaction of Perhaloacetones with Aromatic Hydrocarbons" Seiten 998-1001**

(73) Patentinhaber: **HOECHST AKTIENGESELLSCHAFT**
**Postfach 80 03 20**
**D-6230 Frankfurt am Main 80 (DE)**

(72) Erfinder: **Schneider, Klaus-Albert, Dr.**
**Schillerring 30**
**D-6234 Hattersheim am Main (DE)**
Erfinder: **Siegemund, Günter, Dr.**
**Frankfurter Strasse 21**
**D-6238 Hofheim am Taunus (DE)**

(56) References cited:
**JOURNAL OF FLUORINE CHEMISTRY, Band 24, Nr. 1, 1984 Elsevier Sequoia S.A., Lausanne; J. SEPIOL et al. "Perfluoroalkylation of Benzene derivatives. An improved Synthesis of 1,3-Bis(2-hydroxyhexafluoro-2-propyl)-5-(perfluoro-n-alkyl)benzenes" Seiten 61-74**

**EP 0 254 219 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

1.3- und 1.4-Bis-(2-hydroxyhexafluoro-2-propyl)-benzol sind die Verbindungen der folgenden Formeln (I) und (II):

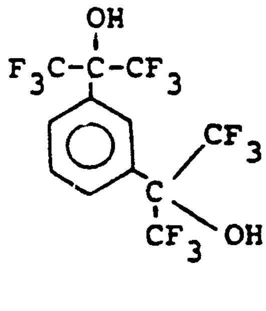

(I)

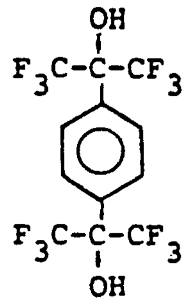

(II)

Sie sind hauptsächlich Zwischenprodukte auf dem Polymerengebiet. Durch Polykondensation z.B. mit Epichlorhydrin

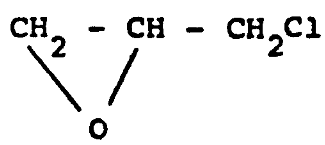

werden wertvolle Epoxidharze von hoher chemischer und thermischer Beständigkeit erhalten.

Die Herstellung von (I) und (II) erfolgt hauptsächlich durch Umsetzung von Benzol mit Hexafluoraceton im Molverhältnis von etwa 1:2 in Gegenwart von $AlCl_3$ als Katalysator; vgl. z.B.

B.S. Farah et al., J. Org. Chem. Vol. 30 (1965), S. 998—1001, sowie

J. Sepiol et al., Journal of Fluorine Chemistry, *24* (1984), S. 61—74.

Bei diesem Herstellverfaren entstehen — wie aus den beiden Literaturstellen hervorgeht — Gemische aus (I) und (II), wobei allerdings der Anteil von (I) denjenigen von (II) erheblich überwiegt (etwa 85—90% (I) und etwa 15 bis 10% (II)).

Die Schmelz- und Siedepunkte von (I) und (II) sind nach den vorerwähnten Literaturstellen sowie nach eigenen Messungen:

| | I | II |
|---|---|---|
| Schmelzpunkt | (bei Raumtemperatur flüssig) | 85—86°C |
| Siedepunkt | 209°C/ 760 mmHg | |
| | 99°C/ 20 mmHg | |
| | 107—112°C/ 24—25 mmHg | 102—105°C 11 mmHg |

Schmelz- und Siedepunkte der beiden Isomeren unterscheiden sich zwar; die Trennung durch fraktionierte Kristallisation ist jedoch wegen der guten Mischkristallbildung nur schwierig möglich. Durch sorgfältige und mehrmalige fraktionierte Destillation sind (I) und (II) zu trennen, doch sind dabei erhebliche Substanzverluste in Kauf zu nehmen.

In der Literaturstelle B.S. Farah et al (a.a.O.) sind keine Angaben über die Trennung der beiden fraglichen Isomeren enthalten. Nach der Literaturstelle von J. Sepiol et al. (a.a.O.) erfolgt z.T. nur eine Anreicherung durch fraktionierte Destillation (Experimental — Teil a auf S. 67/68), z.T. durch Extraktion des mit Wasser versetzten Reaktionsansatzes mit Chloroform, Aufarbeitung der organischen Phase durch Destillation hauptsächlich auf (I) und Kristallisation von (II) aus der wäßrigen Phase im Verlauf mehrerer Tage ("...deposited after several days a crystalline substance" — siehe Experimental — Teil b auf S. 68/69).

Vor allem wegen der langen Kristallisationsdauer ist das letztgenannte Trennverfahren jedoch insbesondere für technische Zwecke kaum geeignet.

In dem Bestreben, ein verbessertes Verfahren zur Trennung von Isomerengemischen aus (I) und (II) bereitzustellen, wurde nun gefunden, daß dieses Ziel dadurch erreicht wird, daß man die Isomerengemische einer Wasserdampfdestillation in gegenwart von Schwefelkohlenstoff $CS_2$ unterwirft, daß man den beim Abkühlen des Destillats ausfallenden — hauptsächlich aus dem 1.4-Isomeren (II)

bestehenden — Feststoff abtrennt, aus dem verbleibenden wäßrig-organischen Flüssigphasengemisch die — hauptsächlich aus dem 1.3-Isomeren (I) bestehende — organische Flüssigphase ebenfalls abtrennt und aus dieser das 1.3-Isomere (I) gewinnt.

Beim Abkühlen des Wasserdampfdetillats fällt hier der — hauptsächlich aus (II) bestehende — Feststoff unverzuğglich aus. Der Nachteil der langen Kristallisationszeiten, wie sie bei den Verfahren von J. Sepiol et al. (a.a.O.) benötigt werden, haftet dem erfindungsgemäßen Verfahren somit nicht an. Außerdem wird durch die Erfindung ein hoher Trenneffekt mit nur geringen Substanzverlusten erzielt. Das Verfahren ist daher wirtschaftlich sehr günstig und auch für die technische Durchführung sehr gut geeignet.

Die Trennung von Gemischen aus (I) und (II) durch eine Wasserdampfdestillation in Gegenwart von $CS_2$ ist sehr überraschen, weil die Isomeren durch eine Wasserdampfdestillation in Abwesenheit von $CS_2$ sowie in Answesenheit anderer Substanzen nicht bzw. nur sehr unvollständig getrennt werden können.

$CS_2$ ist in der Literaturstelle B.S. Farah et al . (a.a.O.) zwar als geeignetes Lösungsmittel für die Umsetzung u.a. von Benzol mit Hexafluoraceton in Gegenwart von $AlCl_3$ als Katalysator beschrieben (vgl. S. 1001, rechte Spalte, Absatz 2); irgendeinen Hinweis über eine mögliche Rolle dieses Lösungsmittels bei der Trennung von (I) und (II) enthält die Literaturstelle jedoch nicht.

Nach dem erfindungsgemäßen Verfahren können im Prinzip Gemische aus (I) und (II) aller möglichen Zusammensetzungen getrennt werden; bevorzugte Ausgangs-Isomerengemische sind jedoch solche aus etwa 10—90% (I) und etwa 90—10% (II), insbesondere solche aus etwa 80—90% (I) und etwa 20—10% (II). In den Rahmen der besonders bevorzugten Ausgangs-Isomerengemische fallen auch die Gemische, wie si normalerweise bei der Umsetzung von Benzol mit Hexafluoraceton im Moverhältnis von etwa 1:2 in Gegenwart von $AlCl_3$ als Katalysator anfallen.

Die erfindungsgemäße Isomerentrennung funktioniert bereits in Gegenwart relativ geringer Mengen $CS_2$. Bevorzugt wird jedoch in Gegenwart von etwa 2,0 bis 6,5 Mol $CS_2$/Mol Isomerengemisch gearbeitet. Das $CS_2$ kann schon bei der Umsetzung von Benzol mit Hexafluoraceton als Lösungsmittel verwendet und für die nachfolgende erfindungsgemäße Wasserdampfdestillation belassen, oder auch erst dem Isomerengemisch vor der erfindungsgemäßen Wasserdampfdestillation zugesetzt werden.

Die Wasserdampfdestillation wird in üblicher Weise — zweckmäßig so lange bis das gesamte Ausgangs-Isomerengemisch überestilliert ist — durchgeführt.

Beim Abkülen des Wasserdampfdestillats fällt normalerweise gleich ein Feststoff aus, welcher von dem verbleibenden wäßrig-organischen Flüssigphasengemisch abgretrennt — zweckmäßig abfiltriert oder abgesaugt — wird. Der Feststoff besteht hauptsächlich aus dem 1,4-Isomeren (II); der Reinheitsgrad liegt meist zwischen etwa 95 und 98%. Z.B. durch Umkristallisation etwa aus Cyclohexan, kann die Reinheit auf über 99% erhöht werden.

Das von dem ausgefallenen Feststoff abgetrennte wäßrigorganische Flüssigphasengemisch wird dann seinerseits in die wäßrige und die organische Phase getrennt. Zwecks vollständiger Entfernung der organischen Bestandteile kann die wäßrige Phase gegebenenfalls noch mit inerten organischen Lösungsmitteln wie z.B. mit aliphatischen Halogenkohlenwasserstoffen ($CH_2Cl_2$, $CHCl_3$, $CCl_4$, $ClCH_2$—$CH_2Cl$, etc.) extrahiert werden. Die organischen Phasen werden vereinigt und wie üblich — zweckmäßig destillativ — aufgearbeitet. Hierbei wird das 1.3-Isomere (I) in hoher Reinheit — bei sorgfältiger Destillation in über 99 %iger Reinheit — erhalten.

Das nachfolgende Beispiel (A) soll das erfindungsgemäße Trennverfahren näher erläutern. Nach diesem Beispiel folgenden noch einige Vergleichsbeispiele (B), welche zeigen, daß die Isomerentrennung durch eine Wasserdampfdestillation in Abwesenheit von $CS_2$ sowie auch in Anwesenheit anderer Substanzen ($CH_2Cl_2$, $CHCl_3$, $CCl_4$, Nitrobenzol, Nitromethan) nicht bzw. nur sehr unvollständig gelingt.

A) Beispiel

a) Herstellung eines Isomerengemisches aus 1.3- aund 1.4- Bis-(2-hydroxyhexafluor-2-propylbenzol) (nicht erfindungsgemäß)

In eine Lösung von 780 g (10 mol) benzol in 3500 ml Schwefelkohlenstoff wurden 3650 g (22 mol) Hexafluoraceton eingeleitet. Die Reaktion wurde mit $AlCl_3$ gestartet sowie bei einer Temperatur von ca. 30—46°C in Gang gehalten. Insgesamt wurden 406 g $AlCl_3$ verbraucht. Der Abgang der Apparatur war durch zwie 1 Meter hohe, mit Paraffin gefüllte "Blasenzähler" sowie einen $CaCl_2$-Turm verschlossen. Nach Raktionsende wurde 12 Stunden nachgerührt, dann bei 0—10°C mit Eiswasser hydrolysiert.

Das Isomerenverhältnis I:II betrug in dem Hydrolysat (lt. GC) 86:14.

b) Erfindungsgemäße Isomerentrennung

Der hydrolysierte Ansatz gemäß a) wurde wasserdampfdestilliert. Der beim Abkühlen des Destillats ausfallende Feststoff wurde durch Absaugen abgetrennt (640 g = 16% Rohausbeute bezogen auf Benzol). Nach der Umkristallisation aus Cyclohexan wurden 432 g 1.4-Isomerers mit einem Schmelzpunkt von 91—94°C erhalten (11% Ausbeute). Die organische Phase des nach dem Absaugen erhaltenen wäßrig-organischen Filtrates wurde abgetrennt und die wäßrige Phase 3 × mit $CH_2Cl_2$ extrahiert. Nach der Trocknung der vereinten organischen Phasen über $MgSO_4$ wurde das Lösungsmittel am Rotationsverdampfer abgezogen und das so erhaltene Rohprodukt über eine kurze Vigreux-Kolonne 15 (25 cm) grob destilliert ($\geqq$ 100°C/25 Torr). Das so vorgerinigte Rohprodukt (2655 g $\hat{=}$ 65% Ausbeute) enthielt das 1.4-Isomere zu 3%, das 1.3-Isomere zu 96—97%.

Über eine 140 cm lange, mit 4 mm Raschig-Ringen gefüllte Kolonne wurde durch eine fraktionierte Vakuum-Destillation das 1.3-Isomere mit einer GC-Reinheit von > 99% erhalten (2301 g ≙ 56% Ausbeute).

Die spektroskopischen und physikalischen Daten stimmen mit denen in der zitierten Literatur überein.

B) Vergleichsbeispiele

a) Herstellung eines Isomerengemisches aus 1.3- aund 1.4- Bis-(2-hydroxyhexafluor-2-propylbenzol)

In 1 Mol Benzol (78 g) ohne weitere Lösungsmittel bzw. in Mischung mit 200 ml eines in der nachstehenden Tabelle enthaltenen Lösungsmittels wurden 2.2-Mol Hexafluoraceton (365 g) eingeleitet. Die Reaktion wurde durch Zugabe von $AlCl_3$ in Gang gebracht und bei 30—46°C aufrecht erhalten. Insgesamt wurden 41 g $AlCl_3$ zugesetzt. Der Abgang der Apparatur war durch zwei 1 m hohe, mit Paraffin gefüllte "Blasenzähler" sowie einen $CaCl_2$-Turm verschlossen. Nach Reaktionsende wurde 12 Stunden nachgerührt, dann bei 0—10°C mit Eiswasser hydrolysiert.

b) Versuch der Isomerentrennung

Der hydrolysierte Ansatz gemäß a) wurde wasserdampfdestilliert. Beim Abkühlen des Destillats ausfallender Feststoff wurde durch Absaugen abgetrennt und getrocknet. Die organische Phase des nach dem Absaugen erhaltenen wäßrig-organischen Filtrats wurde abgetrennt, die wäßrig Phase 3 × mit $CH_2Cl_2$ extrahiert. Nach der Trocknung der vereinten organischen Phasen über $MgSO_4$ wurde $CH_2Cl_2$ und das Lösungsmittel am Rotatinsverdampfer abgezogen und das so erhaltene flüssige Rohprodukt über eine kurze Vigreux-Kolonne (25 cm) grob destilliert (≧ 100°C/25 Torr).

Die Stufen a) und b) wurden unter folgenden Bedingungen mit folgenden Ergebnissen durchgeführt:

| Lösungsmittel | Gesamtausbeute an Isomerengemisch | Ausbeute an isoliertem 1.4-Isomerem nach der Wasserdampfdestillation |
|---|---|---|
| 1. ohne Lösungsmittel | 57% | keine Abtrennung |
| 2. Nitromethan | Spuren | — |
| 3. 1.2-Dichlorethan | 59% | 4% |
| 4. Methylenchlorid | 31% | 3% |

Unabhängig vom Lösungsmittel betrug das 1.3- zu 1.4-Isomerenverhältnis im destillierten Produkt — wie im Rohprodukt vor der Wasserdampfdestillation — 86:14.

**Patentansprüche**

1. Verfahren zur Trennung von Isomerengemischen aus 1.3- und 1.4-Bis-(2-hydroxyhexafluor-2-propyl)-benzol, dadurch gekennzeichnet, daß man die Gemische einer Wasserdampfdestillation in Gegenwart von Schwefelkohlenstoff $CS_2$ unterwirft, daß man den beim Abkülen des Destillats ausfallenden — hauptsächlich aus dem 1.4-Isomeren bestehenden — Feststoff abtrennt, aus dem verbleibenden wäßrig-organischen Flüssigphasengemisch die — hauptsächlich aus dem 1.3-Isomeren bestehende — organische Flüssigphase ebenfalls abtrennt und aus dieser das 1.3-Isomere gewinnt.

2. Verfahren nach Anspruch 1, dadurch gekennzeichnet, daß man als Ausgangs-Isomerengemische solche aus etwa 10—90% des 1.3-Isomeren und etwa 90—10% es 1.4-Isomeren, vorzugsweise solche aus etwa 80—90% des 1.3-Isomeren und etwa 20—10% des 1.4-Isomeren verwendet.

3. Verfahren nach den Ansprüchen 1—2, dadurch gekennzeichnet, daß man als Ausgangs-Isomerengemische die bei der Umsetzung von Benzol mit Hexafluoraceton im Molverhältnis von ca. 1:2 in Gegenwart von $AlCl_3$ als Katalysator erhaltenen Gemische verwendet.

4. Verfahren nach den Ansprüchen 1—3, dadurch gekennzeichnet, daß man die Wasserdampfdestillation in Gegenwart von etwa 2 bis 6.5 Mol $CS_2$ pro Mol Ausgangs-Isomerengemisch, durchführt.

5. Verfahren nach den Ansprüchen 1—4, dadurch gekennzeichnet, daß man aus dem Feststoff, der aus dem Wasserdampfdestillat ausfällt, das reine 1.4-Isomere durch Umkristallisation gewinnt.

6. Verfahren nach den Ansprüchen 1—5, dadurch gekennzeichnet, daß man aus der nach der Wasserdampfdestillation von dem ausgefallenen Feststoff und der wäßrigen Phase abgetrennten organischen Flüssigphase das reine 1.3-Isomere durch Destillation gewinnt.

**Revendications**

1. Procédé pour séparer des mélanges formés par les isomères bis(hydroxy-2 hexafluoro-propyl-2)-1,3 et -1,4 benzènes, procédé caractérisé en ce qu'on soumet les mélanges à une entraînement à la vapeur d'eau effectué en présence de sulfure de carbone $CS_2$,

on sépare le solide précipité lors du refroidissement du distillat et qui consiste essentiellement en l'isomère 1,4,

à partir du mélange restant des phases liquides hydro-organiques, on sépare également la phase liquide organique, consistant essentiellement en l'isomère 1,3, et l'on obtient à partir de cette phase l'isomère 1,3.

2. Procédé selon la revendication 1, caractérisé en ce qu'on utilise comme mélanges d'isomères de départ ceux constitués d'environ 10 à 90% de l'isomère 1,3 et d'environ 90 à 10% de l'isomère 1,4, avantageusement ceux constitués d'environ 80 à 90% de l'isomère 1,3 et d'environ 20 à 10% de l'isomère 1,4.

3. Procédé selon les revendications 1 ou 2, caractérisé en ce qu'on utilise comme mélanges des isomères e départ les mélanges obtenus lors de la réaction du benzène avec l'hexafluoro-acétone, selon un rapport molaire d'environ 1:2, en présence de $AlCl_3$ comme catalyseur.

4. Procédé selon les revendications 1 à 3, caractérisé en ce qu'on conduit l'entraînement à la vapeur d'eau en présence d'environ 2 à 6,5 moles de $CS_2$ par mole du mélange des isomères de départ.

5. Procédé selon les revendications 1 à 4, caractérisé en ce qu'à partir du solide précipté à partir du distillat résultant de l'entraînement à la vapeur d'eau, on obtient par recristallisation l'isomère 1,4 pur.

6. Procédé selon les revendications 1 à 5, caractérisé en ce qu'à partir de la phase liquide organique, séparée du solide, précipité après l'entraînement à la vapeur d'eau, et de la phase aqueuse, on obtient par distillation l'isomère 1,3 pur.

**Claims**

1. A process for separating isomer mixtures of 1,3- and 1,4-bis-(2-hydroxyhexafluoroprop-2-yl)-benzene, which comprises subjecting the mixtures to a steam distillation in the presence of carbon disulfide $CS_2$, separating off the solid which precipitates on cooling of the distillate and is composed mainly of the 1,4-isomer, likewise separating off the organic liquid phase, composed mainly of the 1,3-isomer, from the remaining aqueous-organic liquid-phase mixture and recovering the 1,3-isomer from the organic phase.

2. The process as claimed in claim 1, wherein the starting isomer mixtures used are those of about 10—90% of the 1,3-isomer and about 90—10% of the 1,4-isomer, preferably those of about 80—90% of the 1,3-isomer and about 20—10% of the 1,4-isomer.

3. The process as claimed in claim 1 or 2, wherein the starting isomer mixtures used are those obtained in the reaction of benzene with hexafluoroacetone in a molar ratio of about 1:2 in the presence of $AlCl_3$ as a catalyst.

4. The process as claimed in any of claims 1 — 3, wherein the steam distillation is carried out in the presence of about 2 to 6.5 mol of $CS_2$ per mol of starting isomer mixture.

5. The process as claimed in any of claims 1 — 4, wherein the pure 1,4-isomer is obtained by recrystallization from the solid which precipitates from the steam distillate.

6. The process as claimed in any of claims 1 — 5, wherein the pure 1,3-isomer is obtained by distillation of the organic liquid phase which has been separated after the steam distillation from the precipitated solid and from the aqueous phase.